# EUROPEAN PATENT SPECIFICATION

(11) **EP 1 915 616 B1**
(45) Date of publication and mention of the grant of the patent: **29.02.2012**
(21) Application number: 06765425.1
(22) Date of filing: 12.05.2006
(51) Int. Cl.: G01N 33/53, G01N 33/58

(54) **AN IMMUNO CONJUGATE AND PROCESS FOR PREPARATION THEREOF**
IMMUNKONJUGAT UND VERFAHREN ZUR HERSTELLUNG DAVON
IMMUNO-CONJUGUE ET SON PROCEDE DE PREPARATION

(30) Priority: 26.07.2005 IN DE19832005; 19.10.2005 US 252621
(43) Date of publication of application: 30.04.2008
(73) Proprietor: Council of Scientific and Industrial Research, New Delhi 110 011 (IN)
(72) Inventor: SURI, Chander Raman, Inst. of Microbial Tech., Chandigarh 160 036 (IN); KAUR, Jasdeep, Inst. of Microbial Tech., Chandigarh 160 036 (IN); SINGH, Kanwar Vikas, Inst. of Microbial Tech., Chandigarh 160 036 (IN); VARSHNEY, Girish Chandra, Inst. of Microbial Tech., Chandigarh 160 036 (IN); RAJE, Manoj, Inst. of Microbial Technology, Chandigarh 160 036 (IN)
(74) Representative: Letzelter, Felix Phillip
(86) International application number: PCT/IB2006/001250
(87) International publication number: WO 2007/012926

(56) References cited:
- EP-A2- 0 622 633
- WO-A-97/28451
- US-A- 6 140 134
- WITTMANN C ET AL: "DEVELOPMENT AND EVALUATION OF A DIPSTICK IMMUNOASSAY FORMAT FOR THE DETERMINATION OF ATRAZINE RESIDUES ON-SITE" ANALYST, ROYAL SOCIETY OF CHEMISTRY, LONDON, GB, vol. 121, no. 6, June 1996 (1996-06), pages 863-869, XP008071338 ISSN: 0003-2654 & GIERSCH ET AL: "a new monoclonal antibody for the sensitive detection of atrazine with immunoassay in microtiter plate and dipstick format" J AGRIC FOOD CHEM, vol. 41, 1993, pages 1006-1011, XP002407274
- GASCON JORDI ET AL: "DEVELOPMENT OF A HIGHLY SENSITIVE ENZYME-LINKED IMMUNOSORBENT ASSAY FOR ATRAZINE PERFORMANCE EVALUATION BY FLOW INJECTION, IMMUNOASSAY" ANALYTICA CHIMICA ACTA, ELSEVIER, AMSTERDAM, NL, vol. 347, no. 1-2, 1997, pages 149-162, XP008071358 ISSN: 0003-2670
- LUBAVINA I A ET AL: "A NONINSTRUMENTAL IMMUNOASSAY BASED ON COLLOIDAL DYES" RUSSIAN JOURNAL OF BIOORGANIC CHEMISTRY, MAIK NAUKA-INTERPERIODICA, RU, vol. 26, no. 3, 2000, pages 207-212, XP008071757 ISSN: 1068-1620
- SINGH K V ET AL: "Synthesis and characterization of hapten-protein conjugates for antibody production against small molecules." BIOCONJUGATE CHEMISTRY. 2004 JAN-FEB, vol. 15, no. 1, January 2004 (2004-01), pages 168-173, XP002407254 ISSN: 1043-1802
- HORISBERGER M ET AL: "Labelling of colloidal gold with protein A. A quantitative study", HISTOCHEMISTRY, SPRINGER INTERNATIONAL, DE, vol. 82, no. 3, 1 May 1985 (1985-05-01), pages 219-223, XP008118616, ISSN: 0301-5564, DOI: DOI:10.1007/BF00501398 [retrieved on 2004-12-12]
- OLIVER C: "Conjugation of colloidal gold to proteins", METHODS IN MOLECULAR BIOLOGY, HUMANA PRESS INC, NJ, US, vol. 115, 1 January 1999 (1999-01-01), pages 331-334, XP008118521, ISSN: 1064-3745, DOI: DOI:10.1385/1-59259-213-9:331 [retrieved on 2008-02-02]

## Description

### Field of invention:

The present invention relates to a novel gold-protein-hapten conjugate and process for the preparation of said novel conjugate.

More particularly, it relates to a kit for rapid analyzing a sample for pesticides monitoring using a novel gold-protein-hapten conjugate.

### Background and Prior art:

Pesticides, derived from chemicals, are an essential input in increasing agricultural production by preventing crop losses before and after harvesting. However, their indiscriminate use, apart from being an operational hazard, is posing a serious threat to human health. These organic toxins enter animals and human beings directly or indirectly through the food chain or drinking water. These chemicals being degraded slowly leave behind residues in food and water sources and concentrate as they move up the food chain. Because of their severe toxicity, even at trace levels, it is essential to monitor the levels of these pesticides in the environment, food stuffs, and soil. Due to their recalcitrant and toxic nature, they are listed among priority pollutants. The quick detection of these pollutants is of vital importance for the environmental cleanup. Dipstick based rapid detection of these pollutants has been reported earlier (Giersch, T., J. Agric. Food Chem. 1993, 41: 1006-1011; Mosiello, L. et al., J. Agric. Food Chem. 1998, 46: 3847-3851; Heiss, C, Weller, MG and Niessner, R., Anal. Chim. Acta 1999, 396: 309-316) wherein, Giersch, T. (J. Agric. Food Chem. 1993, 41: 1006-1011) developed a dipstick-based immunoassay using nitrocellulose membrane spotted with goat anti-mouse IgG antibody. In this approach, the strips were treated with specific anti-atrazine antibody (monoclonal antibody, K4E7). The standard atrazine and tracer (atrazine labeled with horseradish peroxidase) solutions were added onto the antibody spots. After washing the membrane with phosphate buffer, the color was developed by treating the strips with tetramethylbenzidine (TMB) substrate. This approach is basically a simple dot-blot based immunoassay where antibody-antigen complex are formed in a solid phase. This approach requires quite a large assay time (5-6 hours) for the complete assay. Similarly, the work of Mosiello, L et al., (J. Agric. Food Chem. 1998, 46: 3847-3851) and Heiss, C, Weller, MG and Niessner, R. (Anal. Chim. Acta 1999, 396: 309-316) also describe the same approach of making dipstick assay in the stationary phase. The drawbacks of the reported methods are that these approaches require very long time for assay (more than 7-8 hours per assay), and also use immuno-reagents, which are not very stable for long-term usages.

WO9728451 discloses the preparation of a gold labeled atrazine carrier conjugate as well as its use in the detection of pesticides in a sample. The preparation involves incubation of atrazine with the antibody carrier protein via carbodiimide chemistry and subsequently gold labeling the carrier protein after adjusting the pH to 7.5 to 7.6 (physisorption)

### Objects of the Invention:

The main object of the present invention is to provide a novel gold-protein-hapten conjugate as defined in the appended claims.

Another object of the present invention is to provide a process for preparing a said novel conjugate as defined in the appended claims.

Yet object of the present invention is to provide a kit of rapid detection of pesticide level from liquid sample by using a novel conjugate as defined in the appended claims.

Still another object of the present invention is to provide a method for detecting and measuring pesticides from water sample by using the said kit as defined in the appended claims.

### Brief description of the Photographs:

In the photograph(s) accompanying this specification

Photograph 1 represents the standard concentration of atrazine in the range between 0 ppb to 1 ppm.

Photograph 2 represents the stability test of developed gold-protein-hapten conjugate

### Summary of the Invention:

The Present invention relates to a kit as defined in the appended claims, based on lateral flow principle where anti pesticide antibody is immobilized at the detection zone on the nitrocellulose membrane while another antibody is coated nearby. The amount of free pesticide present in the water sample competes with the gold protein hapten conjugate to bind with the available limited antibodies binding sites. The color developed due to immuno conjugate is correlated with the concentration of sample. The method is quite simple and specific for the target compound atrazine). The novel gold protein hapten conjugate is highly stable under storage condition at 4°C.

### Detailed description of the Invention:

The present invention is based on the lateral flow of immunoconjugate on a dipstick membrane, is rapid and easy to use for pesticides screening. The conjugate (gold-protein-hapten), which is used as detector reagent in the present invention, is around two fold more stable than the existing protein-gold conjugate used for dipstick applications. The present assay could also be useful in the detection of other toxic compounds such as drugs, heavy metals etc. by using specific anti-analyte antibodies. The stepwise details of the present invention are:
preparation of a novel conjugate,
coating of antibodies on nitrocellulose membrane using standard techniques, development of dipstick format using the conjugate as prepared in step 1, and using a portable reflectometric scanner for semi-quantification of pesticide concentration in sample.

The details of the present invention involve development of a new reagent immunoconjugate for the detection purpose in a dipstick based immunoassay format. For this, a derivative of target pesticide atrazine (mercaptopropionic acid derivative of atrazine) was first synthesized by using standard procedure as reported earlier [K V Singh et al., (2003) J. Anal. Bioanal. Chem.] This was done by adding slowly under constant stirring, a solution of 3-mercaptopropionic acid (5.5 mmole) and 85% KOH (10.8 mmole) made in 10 ml ethanol to a solution of 5.01 mmol atrazine made in 50 ml ethanol. The mixture was refluxed for 6 hours and then the solvent was evaporated under reduced pressure. The residue was taken up in 25 ml 5% NaHCO₃ and washed three times with chloroform. The aqueous layer of the solution was acidified with 6N HCl causing the acidic derivative to precipitate immediately. The supernatant was decanted and the derivative was dried under mild vacuum. The precipitate was further dissolved in ethanol, and then allowed under reduced pressure at 37°C to form mercaptopropionic acid derivative crystals (MPAD). Conjugates of atrazine derivative with protein-gold complex were prepared by mixing protein solution and mercaptopropionic acid derivative of atrazine made in 1 ml dimethylformamide (DMF) along with 125 µmol of dicyclohexyl carbodiimide (DCC) and 125 µmol of N-hydroxysuccinimidyl ester (NHS). The mixture was incubated for 4 h and then centrifuged to remove the urea precipitate. The complex was formed by incubating the protein in the presence of marcaptoethylamine overnight at 4°C, which breaks di-sulfide bonds of the protein so as to link with gold particles to provide a stable complex. The conjugate developed was used in the dipstick format using a nitrocellulose membrane on which anti-pesticide antibody is immobilized at the test line while another antibody (anti-ovalbumin antibody) at the control line on the detection zone. The sample is introduced through the sample pad affixed at one end of the nitrocellulose membrane while on the other end; an absorbance pad is attached to increase the flow of molecules onto the membrane. The sample along with the conjugate move onto the nitrocellulose membrane where these two different molecules react competitively to the available binding sites of the anti-atrazine antibodies coated on the membrane. The intensity of color developed (reversibly) give the presence of analyte in the sample. This was further semi-quantified using a small hand-held reflectometer scanner.

Accordingly the present invention provides a process for preparation of a novel immuno conjugate comprising
**(a)** providing a mercaptopropionic acid derivative of atrazine (Hapten) by known method,
**(b)** Chemisorbing the colloidal gold particles of about 20nm in size to a reduced carrier protein through its thiol groups to obtain the gold protein complex,
**(c)** mixing the complex obtained from step (b) with hapten along with dicyclohexyl carbodiimide (DCC) and N-hydroxysuccinimidyl ester (NHS) dissolved in the solvent Dimethylformamide (DMF),
**(d)** incubating the mixture obtained from step (c) followed by centrifuging to remove the urea precipitate and to obtain the desired immuno conjugate process for preparation of a novel immuno conjugate.

In an embodiment of the present invention, an atrazine derivative hapten is synthesized by known method as described in [K V Singh et al., (2003) J. Anal bioanal. Chem.]

In another embodiment of the present invention, the solution of colloidal gold particles are prepared by heating of about 200ml of 0.01% gold chloride solution to the boiling point.

In yet another embodiment of the present invention, about 4 ml of 1% of tri sodium citrate is added in the boiling solution ogf gold chloride.

In still another embodiment of the present invention, the color of the gold chloride solution is turned immediately to dark purple indicating formation of colloidal gold solution.

In still another embodiment of the present invention, the UV visible spectra showed a peak of colloidal gold solution at 525nm.

In still another embodiment of the present invention, the colloidal gold is chemosorbed to the carrier protein (Ovalbumin) by mixing the reduced ovalbumin to Hapten (mercaptopropionic acid derivative of atrazine).

In still another embodiment of the present invention, the reduced carrier protein solution is formed by incubating the ovalbumin with marcaptoethylamine overnight at about 4°C to break the di-sulfide bonds of the protein so as to link with colloidal gold particles (20nm) to provide stable gold- protein complex.

In still another embodiment of the present invention, the hapten is covalently linked to the protein-gold complex via lysine groups.

In still another embodiment of the present invention, the coupling agents used are dicyclohexyl carbodiimide (DCC) and N- hydroxysuccinimidyl ester (NHS) in ratio of 1:1 dissolved in solvent Dimethylformamide (DMF). at room temperature for about 4 hours.

In still another embodiment of the present invention the mixture is centrifuged at about 10000 rpm for about 5 min to remove the urea precipitate and to obtain a novel immuno conjugate.

Further, the present invention also provides a novel immuno conjugate.

In an embodiment of the present invention, the said immuno conjugate comprises gold, protein hapten in the ratio ranging between 1:25:0.48-1:26:0.5.

In another embodiment of the present invention, the hapten used is mercaptopropionic acid derivatives of atrazine.

In yet another embodiment of the present invention, the protein used is ovalbumin having stock radius about 5nm.

In still another embodiment of the prsent invention, the number of protein molecules per gold particle is ranging between 25-26 by taking protein cover ap. 50% surface area.

In still another embodiment of the prsent invention, the number of hapten per carrier protein molecule is ranging between 12-13 by taking hapten-protein molar ratio 1:50. In still another embodiment of the prsent invention, the said colloidal gold size is about 20nm.

In still another embodiment of the prsent invention, the number of colloidal gold particles per ml are App. 5×10¹².

In still another embodiment of the prsent invention, the available surface area on 20 nm colloidal gold particles is 1256nm².

Present invention also provides a kit for rapid detection of pesticide in a water sample comprising at least one test line, a control line immobilized in detection zone present on or within a support and an immuno conjugate.

In another embodiment of the present invention, an anti ovalbumin is immobilized in a detection zone present on or within a support i.e. nitrocellulose membrane.

In yet another embodiment of the present invention, the water sample contaminated with atrazine is introduced through the sample pad affixed at one end of the nitrocellulose membrane.

In still another embodiment of the present invention, an absorbance pad is attached to increase the flow of molecules onto the membrane:

In still another embodiment of the present invention, the detection of atrazine (pesticide present in water sample) is based on competitive binding of specific anti atrazine antibodies to the immunoconjugate and atrazine sample.

In still another embodiment of the present invention, the atrazine is semi quantified with the help of a small hand held reflectometer.

The present invention further provides a method for detecting and measuring the pesticide from a water a sample.

In an embodiment of the present invention, 50µl of water sample is taken in the sample well.

In another embodiment of the present invention, 50 µl of gold protein hapten conjugate is added in the water sample.

In yet another embodiment of the present invention, the sample along with the conjugate move onto the nitrocellulose membrane where these two different moluecules react competitively to the avaliable binding sites of the anti atrazine antibodies coated on the membrane.

In still another embodiment of the present invention, the wine red color is generated on the test line (coated with anti atrazine antibodies) and control line ( coated with anti ovalbumin antibodies) indicating the presence of pesticide (atrazine).

In still another embodiment of the present invention the colour is detected visually indicating the presence and amount of the pesticide.

In still another embodiment of the present invention a calibration curve in the form of visual presentation is formed which shows the pesticide level in the water sample.

The color intensity of developed hapten-protein-gold conjugate did not show significant loss of intensity even after 6 weeks of its storage time at ambient temperature. This is mainly because of the reason stronger bondformation between gold protein complex made by chemosorbing colloidal gold particles to the reduced albumin through strong Au-S bonds. So this dipstick could successfully be used for around 6-8 weeks time under normal ambient conditions.

The following examples are given by way of illustration and therefore should not be construed to limit the scope of the present invention.

### Example-1

The immuno-conjugate comprising hapten, protein (ovalbumin) and colloidal gold particles were developed for the detection of atrazine in water samples using dipstick based immunoassay format. For this, a solution of colloidal gold particles (20 nm in size) was prepared by heating 200 ml of 0.01% gold chloride solution to boiling point. In the boiling solution of gold chloride, 4ml of 1% trisodium citrate was quickly added. The color of the solution turned immediately to dark purple indicating formation of colloidal gold. UV-visible spectra showed a peak at 525 nm. The colloidal gold was chemisorbed to the carrier protein (ovalbumin) by mixing the reduced ovalbumin to hapten (mercaptopropionic acid derivative of atrazine). The atrazine derivative was synthesized as described earlier [K V Singh et al., (2003) J. Anal. Bioanal. Chem]. Conjugates of this derivative with protein-gold complex were prepared by mixing protein solution and atrazine derivative of atrazine made in 1 ml dimethylformamide along with 125 µmol of dicyclohexyl carbodiimide and 125 µmol of N-hydroxysuccinimidyl ester. The mixture was incubated for 4 h at room temperature and then centrifuged for 5 min at 10000 RPM to remove the urea precipitate. The gold-protein complex was formed by incubating the protein in the presence of marcaptoethylamine overnight at 4°C, which breaks di-sulfide bonds of the protein so as to link with colloidal gold particles (20 nm in size) to provide stable gold-protein complex.

### Example - 2

The development of dipstick format for monitoring pesticides concentration:

The assay is based on the competitive binding of specific anti-atrazine antibodies (bound to nitrocellulose membrane) to the fixed amount of hapten-protein-gold conjugate and atrazine sample. The assay is performed by the application of 50 µl of water sample in the sample well of the dipstick device followed by the addition of 50 µl of above conjugate. The color (wine red) generated on the test line (coated with anti-atrazine antibodies) and control line (coated with anti-ovalbumin antibodies) indicated the presence of atrazine in sample. A hand-held reflectometer scanner at 657 nm was used for the semi-quantification of the test samples. Calibration curve was obtained with the standard atrazine solutions (0 ppb to 5000 ppb), which was further, used for the correlation with atrazine concentration in water sample. The developed approach is very fast and could be very useful for field monitoring.

### Advantages:

The main advantages of the present invention are:
   1. Use of stable immuno-reagents for the fast detection of pesticides.
      Increased shelf life of a novel immunoconjugate (protein-gold hapten conjugate ) means did not show any loss in its activity and sensitivity even after three months of its storage at 4°C.
   2. Rapid screening of pesticide samples.
   3. Semi-quantification of pesticides present in the sample is possible using a less complicated hand-held reflectometer scanner.

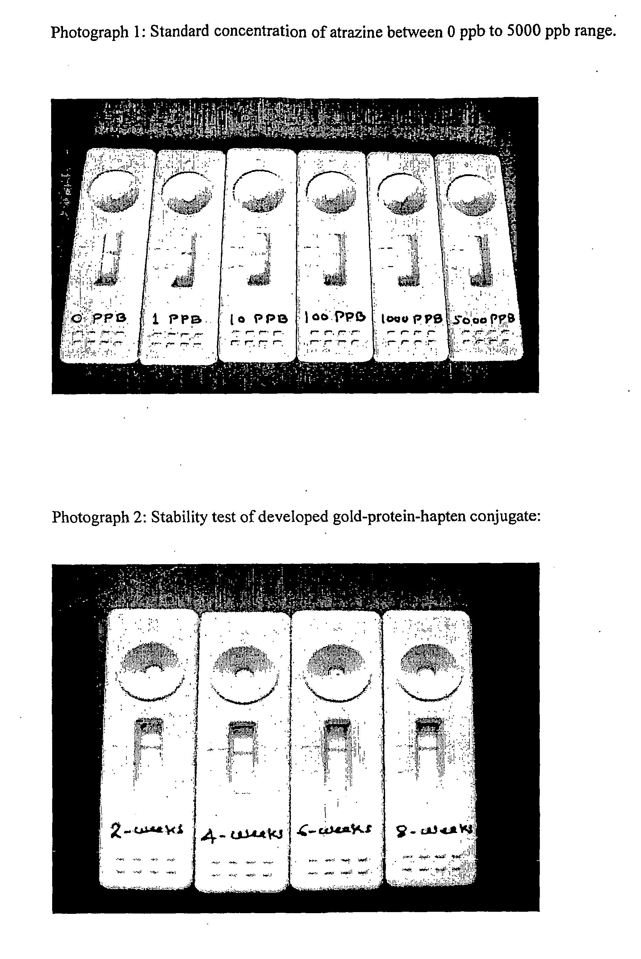

## Claims

1. A process for preparation of a novel immuno conjugate comprising:
a. providing a mercaptopropionic acid derivative of atrazine (Hapten) by known method;
b. chemisorbing the colloidal gold particles of about 20nm in size to a reduced carrier protein through its thiol groups to obtain the gold protein complex;
c. mixing the complex obtained from step (b) with hapten along with dicyclohexyl carbodiimide (DCC) and N-hydroxysuccinimidyl ester (NHS) dissolved in the solvent Dimethylformamide (DMF);
d. incubating the mixture obtained from step (c) followed by centrifuging to remove the urea precipitate and to obtain the desired immuno conjugate.

2. A process as claimed in claim 1, wherein the colloidal gold particles used are prepared by boiling the gold chloride solution in the presence of tri sodium citrate.

3. A process as claimed in claim 1, wherein a reduced carrier protein solution is formed by incubating the ovalbumin with mercaptoethylamine overnight at about 4°C to break the di-sulfide bond of the protein.

4. A process as claimed in claim 1, wherein the hapten used is covalently linked to the said protein-gold conjugate through lysine groups.

5. A process as claimed in claim 1(d), wherein the mixture obtained in step (1c) is centrifuged for a period of about 5 min at about 10000 rpm after incubating the said mixture for about 4 h at room temp to obtain a novel immuno conjugate.

6. A novel immuno conjugate of gold, protein, and a mercaptopropionic acid derivative of atrazine prepared by a process as claimed in claim 1.

7. A novel immuno conjugate as claimed in claim 6, wherein the said immuno conjugate comprises gold, protein hapten in the ratio ranging between 1:25:0.48-1:26:0.5.

8. A novel immuno conjugate as claimed in claim 6, wherein protein used is ovalbumin having stock radius about 5nm.

9. A novel immuno conjugate as claimed in claim 6, wherein number of protein molecules per gold particle is ranging between 25-26 by taking protein cover approximately 50% surface area.

10. A novel immuno conjugate as claimed in claim 6, wherein number of hapten per carrier protein molecule is ranging between 12-13 by taking hapten-protein molar ratio 1:50.

11. A novel immuno conjugate as claimed in claim 6, wherein the said colloidal gold size is about 20nm.

12. A novel immuno conjugate as claimed in claim 6, wherein number of colloidal gold particles per ml are approximately 5x10¹².

13. A novel immuno conjugate as claimed in claim 6, wherein available surface area on 20 nm colloidal gold particles is 1256nm².

14. A kit for rapid detection of atrazine in a water sample comprising at least one test line, a control line immobilized in a detection zone present on or within a support and an immuno conjugate according to at least one of the claims 6 to 13.

15. A kit as claimed in claim 14, wherein the test line used is an anti-atrazine antibody.

16. A kit as claimed in claim 14, wherein the control line used is an anti-ovalbumin antibody.

17. A kit as claimed in claim 14, wherein the said kit further comprising a reflectometer scanner.

18. A kit as claimed in claim 14, wherein the support used is nitrocellulose.

19. A kit as claimed in claim 14, wherein the detection is based on competitive binding of specific anti-atrazine antibodies to the immuno conjugate and atrazine sample.

20. A method for detecting and measuring pesticide in a sample comprising contacting the kit of claim 14 with a sample and detecting and/or measuring the pesticide level.

21. A method as claimed in claim 20, wherein the contacting of sample with the kit of claim 15 generates colour in the test line.

22. A method as claimed in claim 20, wherein the colour generated is wine red.

23. A method as claimed in claim 20, wherein the colour is detected visually indicating the presence and amount of the pesticide.

## Patentansprüche

1. Verfahren zur Herstellung eines neuen Immunkonjugats, umfassend:
a. Bereitstellen eines Mercaptopropionsäure-Derivats von Atrazin (Hapten) durch ein bekanntes Verfahren;
b. Chemisorbieren von kolloidalen Goldpartikeln mit einer Größe von etwa 20 nm an ein reduziertes Trägerprotein durch seine Thiol-Gruppen unter Erhalt des Gold-Protein-Komplexes;
c. Mischen des aus Schritt (b) erhaltenen Komplexes mit Hapten zusammen mit Dicyclohexylcarbodiimid (DCC) und N-Hydroxysuccinimidylester (NHS), gelöst in dem Lösungsmittel Dimethylformamid (DMF);
d. Inkubieren des aus Schritt (c) erhaltenen Gemisches, gefolgt von Zentrifugieren, um das Harnstoffpräzipitat zu entfernen und das gewünschte Immunkonjugat zu erhalten.

2. Verfahren, wie es in Anspruch 1 beansprucht ist, wobei die kolloidalen Goldpartikel, die verwendet werden, durch Sieden einer Goldchloridlösung in Gegenwart von Trinatriumcitrat hergestellt werden.

3. Verfahren, wie es in Anspruch 1 beansprucht ist, wobei eine Lösung von reduziertem Trägerprotein gebildet wird, indem das Ovalbumin über Nacht mit Mercaptoethylamin bei etwa 4 °C inkubiert wird, um die Disulfidbindung des Proteins aufzubrechen.

4. Verfahren, wie es in Anspruch 1 beansprucht ist, wobei das verwendete Hapten kovalent durch Lysin-Gruppen mit dem Protein-Gold-Konjugat verknüpft wird.

5. Verfahren, wie es in Anspruch 1 (d) beansprucht ist, wobei das Gemisch, das in Schritt (1c) erhalten wird, für einen Zeitraum von etwa 5 min bei etwa 10000 Upm nach Inkubieren des Gemisches für etwa 4 h bei Raumtemperatur zentrifugiert wird, um ein neues Immunkonjugat zu erhalten.

6. Neues Immunkonjugat aus Gold, Protein und einem Mercaptopropionsäurederivat von Atrazin, das durch ein Verfahren, wie es in Anspruch 1 beansprucht ist, hergestellt wird.

7. Neues Immunkonjugat, wie es in Anspruch 6 beansprucht ist, wobei das Immunkonjugat Gold, Protein, Hapten in einem Verhältnis im Bereich von 1:25:0,48 bis 1:26:0,5 umfasst.

8. Neues Immunkonjugat, wie es in Anspruch 6 beansprucht ist, wobei das verwendete Protein Ovalbumin ist, das einen Stoffradius (stock radius) von etwa 5 nm hat.

9. Neues Immunkonjugat, wie es in Anspruch 6 beansprucht ist, wobei die Anzahl an Proteinmolekülen pro Goldpartikel im Bereich zwischen 25-26 liegt, wobei angenommen wird, dass das Protein etwa 50 % Oberflächenbereich bedeckt.

10. Neues Immunkonjugat, wie es in Anspruch 6 beansprucht ist, wobei die Anzahl von Hapten pro Trägerproteinmolekül im Bereich zwischen 12-13 liegt, wobei ein Hapten-Protein-Molverhältnis von 1:50 genommen wird.

11. Neues Immunkonjugat, wie es in Anspruch 6 beansprucht ist, wobei die Größe des kolloidalen Golds etwa 20 nm ist.

12. Neues Immunkonjugat, wie es in Anspruch 6 beansprucht ist, wobei die Anzahl an kolloidalen Goldpartikeln pro ml etwa 5x10¹² ist.

13. Neues Immunkonjugat, wie es in Anspruch 6 beansprucht ist, wobei der verfügbare Oberflächenbereich auf kolloidalen 20 nm-Goldpartikeln 1256 nm² ist.

14. Kit zur schnellen Detektion von Atrazin in einer Wasserprobe, umfassend wenigstens eine Testlinie, eine Kontrolllinie, immobilisiert in einer Detektionszone, die an oder in einem Träger vorliegt, und ein Immunkonjugat gemäß wenigstens einem der Ansprüche 6 bis 13.

15. Kit, wie er in Anspruch 14 beansprucht ist, wobei die verwendete Testlinie ein Anti-Atrazin-Antikörper ist.

16. Kit, wie er in Anspruch 14 beansprucht ist, wobei die verwendete Kontrolllinie ein Anti-Ovalbumin-Antikörper ist.

17. Kit, wie er in Anspruch 14 beansprucht ist, wobei der Kit außerdem einen Reflektometer-Scanner umfasst.

18. Kit, wie er in Anspruch 14 beansprucht ist, wobei der verwendete Träger Nitrocellulose ist.

19. Kit, wie er in Anspruch 14 beansprucht ist, wobei die Detektion auf kompetitive Bindung von spezifischen Anti-Atrazin-Antikörpern an das Immunkonjugat und die Atrazinprobe basiert.

20. Verfahren zum Detektieren und Messen von Pestizid in einer Probe, das In-Kontakt-Bringen des Kits gemäß Anspruch 14 mit einer Probe und Detektieren und/oder Messen des Pestizidlevels umfasst.

21. Verfahren, wie es in Anspruch 20 beansprucht ist, wobei das In-Kontakt-Bringen von Probe mit dem Kit gemäß Anspruch 15 Farbe in der Testlinie erzeugt.

22. Verfahren, wie es in Anspruch 20 beansprucht ist, wobei die erzeugte Farbe Weinrot ist.

23. Verfahren, wie es in Anspruch 20 beansprucht ist, wobei die Farbe visuell detektiert wird, was das Vorliegen und die Menge des Pestizids anzeigt.

## Revendications

1. Procédé pour la préparation d'un immunoconjugué nouveau, comprenant les étapes consistant à :
a. fournir un dérivé d'acide mercaptopropionique d'atrazine (haptène) par un procédé connu ;
b. soumettre à une chimisorption les particules d'or colloïdal d'environ 20 nm de diamètre à une protéine de support réduite par l'intermédiaire de ses groupes thiol pour obtenir le complexe or-protéine ;
c. mélanger le complexe obtenu dans l'étape (b) avec l'haptène conjointement avec du dicyclohexylcarbodiimide (DCC) et de l'ester de N-hydroxysuccinimidyle (NHS) à l'état dissous dans un solvant consistant en diméthylformamide (DMF) ;
d. mettre en incubation le mélange obtenu dans l'étape (c) puis effectuer une centrifugation pour éliminer le précipité d'urée et pour obtenir l'immunoconjugué désiré.

2. Procédé suivant la revendication 1, dans lequel les particules d'or colloïdal utilisées sont préparées par ébullition de la solution de chlorure d'or en présence de citrate trisodique.

3. Procédé suivant la revendication 1, dans lequel une solution de protéine de support réduite est formée par mise en incubation de l'ovalbumine avec de la mercapto-éthylamine pendant une nuit à environ 4°C pour la rupture de la liaison disulfure de la protéine.

4. Procédé suivant la revendication 1, dans lequel l'haptène utilisé est lié par covalence audit conjugué protéine-or par des groupes lysine.

5. Procédé suivant la revendication 1(b/d), dans lequel le mélange obtenu dans l'étape 1(c) est centrifugé pendant une période d'environ 5 min à environ 10 000 tr/min après mise en incubation dudit mélange pendant environ 4 h à température ambiante pour obtenir un immunoconjugué nouveau.

6. Immunoconjugué nouveau d'or, d'une protéine et d'un dérivé d'acide mercaptopropionique d'atrazine préparé par un procédé suivant la revendication 1.

7. Immunoconjugué nouveau suivant la revendication 6, ledit immunoconjugué comprenant de l'or, une protéine et un haptène en un rapport allant de 1:25:0,48 à 1:26:0,5.

8. Immunoconjugué nouveau suivant la revendication 6, dans lequel la protéine utilisée est l'ovalbumine ayant un rayon de Stokes d'environ 5 nm.

9. Immunoconjugué nouveau suivant la revendication 6, dans lequel le nombre de molécules de protéine par particule d'or va de 25 à 26 en considérant un recouvrement de la protéine d'approximativement 50 % de la surface.

10. Immunoconjugué nouveau suivant la revendication 6, dans lequel le nombre de molécules d'haptène par molécule de protéine de support va de 12 à 13 en considérant un rapport molaire haptène-protéine de 1:50.

11. Immunoconjugué nouveau suivant la revendication 6, dans lequel le diamètre dudit or colloïdal est d'environ 20 nm.

12. Immunoconjugué nouveau suivant la revendication 6, dans lequel le nombre de particules d'or colloïdal par ml est d'approximativement 5 x 10¹².

13. Immunoconjugué nouveau suivant la revendication 6, dans lequel la surface disponible sur les particules d'or colloïdal de 20 nm est de 1256 nm².

14. Kit pour la détection rapide d'atrazine dans un échantillon d'eau, comprenant au moins une ligne d'essai, une ligne témoin immobilisée dans une zone de détection présente sur un ou à l'intérieur d'un support et un immunoconjugué suivant au moins une des revendications 6 à 13.

15. Kit suivant la revendication 14, dans lequel la ligne d'essai utilisée est constituée d'un anticorps anti-atrazine.

16. Kit suivant la revendication 14, dans lequel la ligne témoin utilisée est constituée d'un anticorps anti-ovalbumine.

17. Kit suivant la revendication 14, ledit kit comprenant en outre un scanner de réflectomètre.

18. Kit suivant la revendication 14, dans lequel le support utilisé est constitué de nitrocellulose.

19. Kit suivant la revendication 14, dans lequel la détection est basée sur la liaison compétitive d'anticorps anti-atrazine spécifiques à l'immunoconjugué et à l'échantillon d'atrazine.

20. Procédé pour détecter et doser un pesticide dans un échantillon, comprenant la mise en contact du kit de la revendication 14 avec un échantillon et la détection et/ou la mesure eu taux du pesticide.

21. Procédé suivant la revendication 20, dans lequel la mise en contact de l'échantillon avec le kit de la revendication 15 engendre une couleur dans la ligne d'essai.

22. Procédé suivant la revendication 20, dans lequel la couleur engendrée est la couleur rouge vineux.

23. Procédé suivant la revendication 20, dans lequel la couleur est détectée visuellement, ce qui indique la présence et la quantité du pesticide.
